# EUROPÄISCHE PATENTANMELDUNG

(11) **EP 4 111 999 A1**
(43) Veröffentlichungstag der Anmeldung: **04.01.2023**
(21) Anmeldenummer: 21183281.1
(22) Anmeldetag: 01.07.2021
(51) Int. Cl.: A61B 90/00, A61C 5/82, A61C 17/10, A61G 10/00, A61G 13/10, A61B 90/40, A61B 1/24, A61C 5/90, A61C 17/06

(54) **ABSAUGRING**

(71) Anmelder: Ivoclar Vivadent AG, 9494 Schaan (LI)
(72) Erfinder: LICHTENSTEIGER, Markus, 9462 Montlingen (CH)
(74) Vertreter: Baronetzky, Klaus

(57) **Zusammenfassung**

Die Erfindung betrifft einen Absaugring für die Anbringung nahe der Mundöffnung eines zahnärztlichen Patienten, mit einem extraoralen Spannelement. Das Spannelement ist als Saug-Spannelement realisiert und bildet mit einem Folienelement einen Unterdruckraum aus oder aufspannt diesen auf. Der Unterdruckraum erstreckt sich ringförmig oder teilringförmig, und der Unterdruckraum weist mindestens eine, insbesondere radial einwärts weisende, Saugdüse auf.

## Beschreibung

Die vorliegende Erfindung betrifft einen Absaugring gemäß dem Oberbegriff von Anspruch 1 bzw. 16.

Zahnärztliche Vorrichtungen sind seit langem bekannt. Hierzu gehören insbesondere speichelsauger. Es ist aber auch seit langem bekannt, Aerosole aus dem Mundbereich des Patienten abzusaugen. Mit einer entsprechenden Absaugvorrichtung soll eine Infektion des Zahnarzt durch den Patienten erreicht werden, aber auch die keim- und Virenfreiheit im Behandlungsraum verbessert werden.

Ein Beispiel für eine derartige Saugvorrichtung lässt sich der DE 37 26 394 A1 entnehmen. Bei dieser Aerosolabsaugvorrichtung wird eine Saugdüse im Mundbereich des Patienten angebracht und, und die durch die und in der Atemluft des Patienten vorliegenden Aerosole können abgesaugt und getrennt werden.

Ferne ist es bereits vorgeschlagen worden um, eine Unterdruckquelle in der Nähe des Keime oder Viren abgebenden Menschen vorzusehen. Hierdurch soll aus der Umgebung keimfreie Frischluft zugeführt werden, so dass Infektionen unterbleiben sollen. Nachteil einer solchen Lösung ist, dass nicht sichergestellt ist, dass der Mensch nicht doch andere Menschen infiziert, insbesondere solche, die in der Nähe seiner Mundöffnung sind.

Daher liegt der Erfindung die Aufgabe zugrunde, einen Absaugring gemäß dem oberbegriff von Anspruch 1 bzw. 16 zu schaffen, der eine verbesserte Infektionssicherheit bietet, aber dennoch einfach zu handhaben ist.

Diese Aufgabe wird erfindungsgemäß durch Anspruch 1 bzw. 16 gelöst. Vorteilhafte Weiterbildungen ergeben sich aus den Unteransprüchen.

Erfindungsgemäß ist es vorgesehen, einen Absaugring für Aerosole bereitzustellen, der unmittelbar an der Mundöffnung eines Patienten angebracht werden kann. Die Mundöffnung stellt regelmäßig aus infektiologischer Sicht den Infektionsherd dar, insbesondere, was die Abgabe von Aerosolen angeht.

Erfindungsgemäß wird der absaugring, was seinen Unterdruckraum angeht, unter Unterdruck gesetzt. Der Unterdruckraum erstreckt sich etwas außerhalb der Mundöffnung und setzt den Bereich der Mundöffnung über eine Saugdüse unter Unterdruck. Hierdurch ist sichergestellt, dass vom Patienten abgegebene Keime oder Viren unmittelbar nach Abgabe aus dem Mund abgesaugt und damit unschädlich gemacht werden.

Bevorzugt erstreckt sich die Saugdüse radial einwärts. Es ist aber auch möglich, dass sie sich in die Mundöffnung hinein erstreckt oder den Bereich vor der Mundöffnung absaugt und dementsprechend dort endet.

Unter Absaugring oder Saugring sei hier ein beliebiges insbesondere gekrümmtes Element zu verstehen und, dass mindestens als teilringförmig angesehen werden kann. Dies bedeutet, dass kein geschlossener Ring also einen vollring und, vorliegen muss, um die erwünschte Wirkung zu erzielen. Es ist auch möglich um, eine Absaugung an zwei um den Umfang verteilten Stellen an der Mundöffnung des Patienten vorzunehmen, oder aber, an mehreren um den Umfang verteilten Stellen.

Der erfindungsgemäße Absaugring hat bevorzugt solche Abmessungen, dass er extraoral auf den Lippen des Patienten aufliegen kann, auch wenn dieser seinen Mund geöffnet hat. Damit ergibt sich ein Querschnitt oder Durchmesser von etwa 5 bis 10 cm.

In erfindungsgemäß besonders günstigerweise ist der absaugring mit einem Spannelement und einem Folienelement versehen. Das Folienelement ist mit dem Spannelement verbunden. Das Folienelement durchtritt in an sich bekannter Weise bevorzugt die Mundöffnung des Patienten. Damit ist das Spannelement vor der Mundöffnung des Patienten gelagert Punkt das Spannelement hat eine Doppelfunktion, indem es nämlich zum einen das Folienelement aufspannt und zum anderen den Unterdruckraum bereitstellt oder aufspannt.

Das folienelement legt sich durch die Aufspannwirkung des Spannelement an die Lippen des Patienten an, sodass auch eine Berührung zwischen den Lippen des Patienten und den Händen des Zahnarzt sicher ausgeschlossen ist. Diese Funktion ist von den sehr erfolgreichen Lippen/Wangen-Haltern "Optragate" der vorliegenden Anmelderin bekannt. Ein derartiger Halter weist ein intraorales Spannelement und ein extraorales Halter-Spannelement auf, zwischen denen sich das Folienelement erstreckt. Ein solcher Halter lässt sich erfindungsgemäß gut einsetzen.

Das erfindungsgemäße Spannelement ist entweder als besonderes extraorales Saug-Spannelement mit dem Folienelement unmittelbar verbunden.

Es ist aber auch möglich an, einen Standard-Halter und, beispielsweise den Halter "Optragate" einzusetzen. An dessen extraorales Halter-Spannelement wird dann das erfindungsgemäße extraorale Saug-Spannelement angebracht. Die Anbringung kann in beliebiger geeigneter Weise erfolgen, beispielsweise, indem das Formteil, das das erfindungsgemäße Spannelement bildet, angeklipst oder aufgeklipst oder angeklemmt oder aufgeklemmt an dem Halter-Spannelement gelagert ist. Es ist eine beliebige geeignete Verbindung möglich. Bevorzugt ist eine solche Verbindung, die frei von Hinterschneidungen ist und insbesondere keine Innenecken an dem erfindungsgemäßen Saug-Spannelement erzeugt.

Typischerweise ist das Halter-Spannelement elastischer als das erfindungsgemäße Saug-Spannelement. Es bietet sich daher an, die Lagerung durch eine elastische Verformung des Halter-Spannelement bereitzustellen, das dementsprechend unter Vorspannung auf das Saug-Spannelement aufgeschoben oder in dieses eingeschoben wird und dort festklemmt.

Diese Lösung hat den Vorteil, dass der erfindungsgemäße Absaugring an Standard-Lippen/Wangen-Haltern mehrfach verwendbar ist bzw. dass solche Halter mit einem erfindungsgemäßen Absaugring nachrüstbar sind.

Sowohl der erfindungsgemäße Unterdruckraum als auch die erfindungsgemäße Saugdüse können in beliebiger geeigneter Weise ausgestaltet sein. Der Unterdruckraum ist mit einem Sauganschluss ausgestattet. Über diese wird er mit einer in Zahnarztpraxen regelmäßig vorliegenden Unterdruckquelle verbunden. Bevorzugt ist der Unterdruckraum in einem Kunststoffkörper, der beispielsweise in Form eines Spritzgussteils vorliegt, ausgebildet.

Der Unterdruckraum kann aber auch von einem oder mehreren Spannelementen aufgespannt gehalten sein, die von dem Folienelement abgedeckt sind oder zwischen denen sich ein Folienelement erstreckt. Das Folienelement kann ein separates Folienelement sein, das für die Ausbildung des Unterdruckraums bestimmt ist, oder es kann einstückig mit dem Folienelement des Lippen/Wangen-Halters sein, diesen also insofern extraoral verlängern.

Erfindungsgemäß besonders günstig ist es, dass das Spannelement das Folienelement unter Spannung hält. Das Folienelement des Lippen/Wangen-Halters verläuft durch die Mundöffnung hindurch und bedeckt die Lippen des Patienten. Durch die Vorspannung des Spannelements wird das Folienelement dort glatt gezogen, so dass sich keine Falten h#ergeben. Es versteht sich, dass das extraorale Spannelement insofern bevorzugt mit einem intraoralen Spannelement zusammenwirkt, wie es bei dem "Optragate" an sich bekannt ist. Bei dieser Ausgestaltung verbindet das Folienelement das extraorale Spannelement mit dem intraoralen Spannelement.

Das Folienelement ist in an sich bekannter Weise im wesentlichen schlauchförmig, mit eine mittigen, einwärts gewölbten Einschnürung. Es ist bevorzugt Teil des Lippen/Wangen-Halters, der auch ein kreisringförmiges oder ovales intraorales Spannelement aufweist. Dieser kann auch als intraoraler Spannring bezeichnet werden. Er dient dazu, das Folienelement intraoral im Vestibulum aufgespannt zu halten, so dass das Folienelement an den Lippen des Patienten vollflächig anliegt.

Bei einem bekannten Lippen/Wangen-Halter ist zudem ein extraorales Spannelement oder ein extraoraler Spannring vorgesehen, der das Folienelement in gleicher Weise wie das intraorale aufgespannt hält. Dieses kann in günstiger Weise als Lagerbasis des Ansaugrings verwendet werden. Hierzu wird das Saug-Spannelement an oder auf dem extraoralen Spannring oder -element gelagert. Die Lagerung kann durch eine Klemmverbindung, durch eine Spannverbindung, also eine solche, bei dem das Saug-Spannelement den extraoralen Spannring übergreift, untergreift oder hintergreift und diese sich gegenseitig unter Spannung setzen, oder durch eine Klipsverbindung erfolgen.

Besonders günstig ist es alternativ, wenn das Saug-Spannelement das Folienelement im gespannten Zustand hält. Hierdurch ist es möglich, auf ein zusätzliches extraorales Spannelement des Folienelementes zu verzichten.

Das Spannelement hält das Folienelement im gespannten Zustand, und wird durch das Folienelement selbst unter Spannung gesetzt.

Das kreisförmige oder ovale Saug-Spannelement hat einen größeren Durchmesser als das Folienelement in entspanntem Zustand. Durch eine hierauf basierende Spannverbindung zwischen diesen, bei der das z.B. das Folienelement das Saug-Spannelement übergreift, wird sowohl das Saug-Spannelement unter eine radial einwärts weisende Spannung gesetzt, als auch das Folienelement unter eine radial auswärts weisende Spannung gesetzt. Dies ermöglicht eine sichere, aber bei Bedarf lösbare Verbindung.

Der erfindungsgemäße Absaugring ist bevorzugt für den Anschluss an einen Lippen/Wangen-Halter ausgebildet oder einstückig mit diesem. Dieser weist das Folienelement auf. Das Folienelement ist für die Erstreckung über die Lippe eines Patienten ausgebildet ist, an welcher Stelle das Folienelement ein Abdeckelement bildet. Insofern ist das Saug-Spannelement als extraorales Saug-Spannelement bevorzugt so ausgebildet, dass es über ein Abdeckelement als Folienelement mit dem intraoralen Spannelement verbunden ist.

In vorteilhafter Ausgestaltung ist es vorgesehen, dass das Saug-Spannelement Aufnahmemittel für das Folienelement aufweist, über welche es mit dem Folienelement, insbesondere lösbar, verbunden oder verbindbar ist. Besonders günstig ist eine lösbare Verbindung. Das Saug-Spannelement kann als fester Kunststoffkörper ausgebildet sein. Dieser nimmt den Unterdruckraum auf. Über die Aufnahmemittel lässt sich das Folienelement an dem Saug-Spannelement befestigen.

Nach Gebrauch wird es gelöst und entsorgt, und das Saug-Spannelement kann für die nächste Versorgung verwendet werden.

Die Aufnahmemittel können auch so ausgebildet sein, dass sie einen Lippen/Wangen-Halter lagern können. Dieser weist dann seinerseits das Folienelement auf, so dass eine indirekte Lagerung dieses durch die Aufnahmmittel des Saug-Spannelements realisiert ist.

Weitere Vorteile, Einzelheiten und Merkmale ergeben sich aus der nachfolgenden Beschreibung mehrerer Ausführungsbeispiele der Erfindung anhand der Zeichnung.

Es zeigen:
- Fig. 1: eine perspektivische Ansicht aus einer Ausführungsform eines erfindungsgemäßen Absaugrings;
- Fig. 2: eine perspektivische Ansicht des Absaugrings gemäß Fig. 1, mit dem ein Folienelement verbunden ist;
- Fig. 3: ein vergrösserter Schnitt durch die Ausführungsform gemäß den Fig. en 1 und 2, wobei der Unterdruckraum und das Aufnahmemittel für die Verbindung des Folienelements mit dem Spannelement dargestellt sind;
- Fig. 4: eine schematisierte Darstellung einer Anbringung eines erfindungsgemäßen Absaugrings, angebracht an einem Patienten;

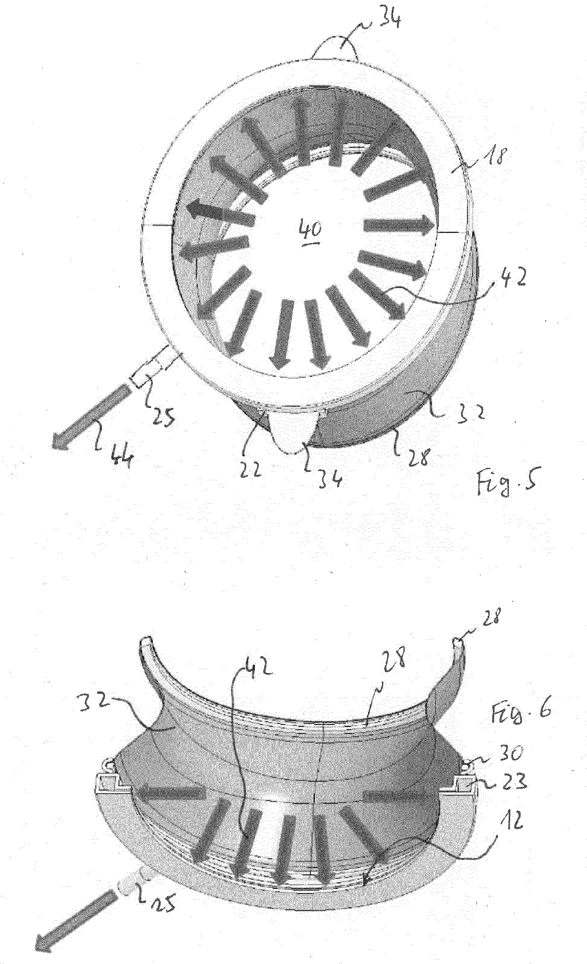

- Fig. 5: eine schematisierte perspektivische Darstellung der Absaugungen durch den erfindungsgemäßen Absaugring;
- Fig. 6: die Ausführungsform gemäß Fig. 5 in einer anderen Darstellung und unter teilweiser Entfernung eines Teils des Absaugrings;
- Fig. 7: eine schematische Schnittdarstellung durch eine weitere Ausführungsform eines erfindungsgemäßen Absaugrings mit integriertem Lippen/Wangen-Halter, mit einem Folienelement, das einstückig mit dem Absaugring im übrigen ist;
- Fig. 8: eine schematische Draufsicht auf den Absaugring in der Ausführungsform gemäß Fig. 7, mit einem sich spiralig erstreckende Spannelement;
- Fig. 9: eine schematische Darstellung einer weiteren Ausführungsform eines erfindungsgemäßen Absaugrings in einer zweiteiligen Ausgestaltung mit dem Lippen/Wangen-Halters; und
- Fig. 10: eine gegenüber Fig. 9 modifizierte Ausgestaltung des Absaugrings unter Verwendung des gleichen Lippen/Wangen-Halters.

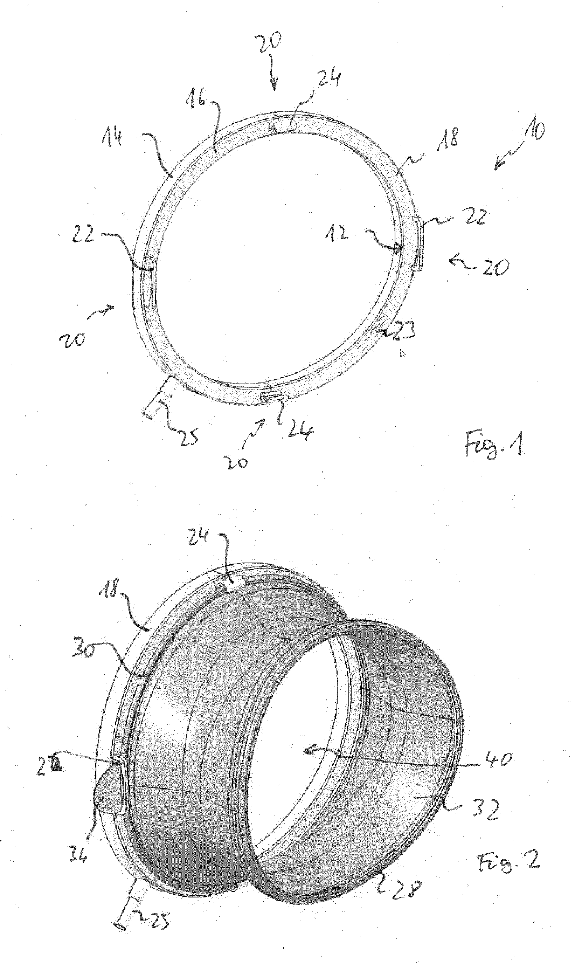

Aus Fig. 1 ist eine perspektivische Darstellung eines erfindungsgemäßen Absaugrings 10 ersichtlich. In der Darstellung gemäß Fig. 1 ist der Absaugring 10 kreisringförmig und als Hohlprofil ausgebildet. Er besteht aus zwei aufeinander geklipsten Spritzguss-Formteilen aus Kunststoff und weist an seiner radial inneren Seite einen Schlitz oder Spalt auf, der eine Saugdüse 12 bildet.

Der Absaugring 10 ist in Fig. 1 in einem das Saug-Spannelement 18 bildenden Teil dargestellt. Das Saug-Spannelement 18 weist Aufnahmemittel 20 auf. Hierzu gehören zwei Ösen 22 und zwei Klammern 24.

Das Saug-Spannelement 18 spannt einen Unterdruckraum 23 auf, der mit der Saugdüse 12 verbunden ist. Der Unterdruckraum 23 erstreckt sich ringförmig oder teilringförmig, Der Unterdruckraum 23 wird seinerseits über einen Sauganschluss 25 unter Unterdruck gesetzt, denn dieser ist über einen nicht dargestellten Schlauch mit einer nicht dargestellten Unterdruckquelle verbunden.

Die Saugdüse 12 ist als Ringspalt ausgebildet und saugt dementsprechend Luft und etwaige dort befindliche Aerosole aus der Mitte des Saug-Spannelements 18 an. Diese werden im Saug-Spannelement 18 gesammelt und über den Sauganschluss 25 abgeführt. Durch die Ausrichtung der Saugdüse 12 nach radial eimwärts wird die Absaugung aus der Mitte des Spannelements 18 besonders begünstigt.

Die Aufnahmemittel 20 dienen der Lagerung eines in Fig. 2 dargestellten Lippen/ Wangen-Halters 26. Der Halter 26 weißt ein intraorales Spannelement 28 und ein extraorales Spannelement 30 auf, zwischen denen sich ein Folienelement 32 erstreckt. Mittels der Aufnahmemittel 20 ist der Halter 26 an dem Saug-Spannelement 18 gelagert.

Hierbei umgreift die Klammer 24 das extraorale Spannelement 30 von außen. In die Öse 22 ist ein nach außen vorspringender Spannlappen 34 des Halters 26 eingesteckt und lagert dieses. Der Spannlappen 34 besteht aus Folienmaterial wie das Folienelement 32.

Der extraorale Spannring 30 ist unter Vorspannung in den Saug-Spannring 18 eingesetzt. Der Spannring 30 wird durch die Wirkung der Klammern 24 etwas zusammengedrückt.

Der Halter 26 ist in einer an sich bekannten Weise wie ein "Optragate" ausgebildet. Die Spannelemente 30 und 28 sind im Wesentlichen kreisförmig und aus elastischen Kunststoffringen. Zwischen ihnen ist das Folienelement 32 aufgespannt. In der Mitte zwischen den Ringen 28 und 30 ist es nach radial einwärts eingewölbt. Diese Stelle ist für die Lippenauflage bestimmt. Das aus einem sehr elastischen Material bestehende Folienelement 32 schmiegt sich an die Lippen an und erstreckt sich sowohl intraoral als auch extraoral an diesen entlang.

Durch die Öffnung 40 in dem Halter 26 und in dem Saug-Spannring 18 kann der Patient auch bei eingesetztem Absaugring 10 atmen, und der Zahnarzt kann die zahnärztlichen Behandlungen durch die Öffnung 40 hindurch durchführen.

Beim Einschalten der Unterdruckquelle wird zugleich die Öffnung 40 abgesaugt. Atemluft sowohl des Patienten als auch gegebenenfalls des Zahnarztes, und Aerosole werden zuverlässig abgesaugt, so dass weder der Patient noch der Zahnarzt durch die Atemluft des jeweils anderen kontaminiert werden.

Damit werden beide vor Infektionen geschützt, unabhängig davon, ob beispielsweise Keime oder Viren in der Atemluft vorliegen.

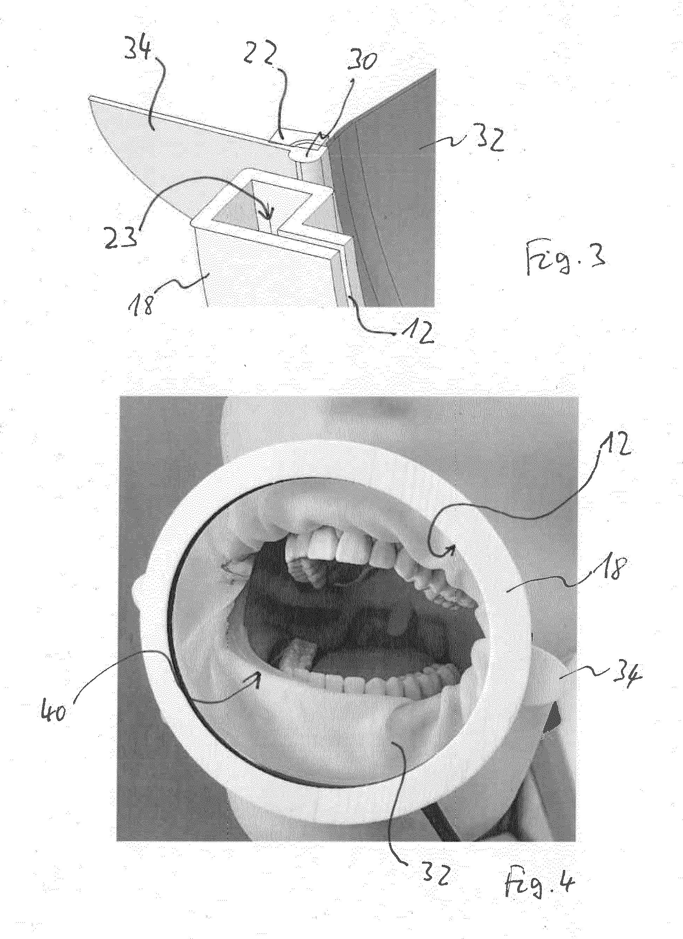

Eine vergrößerte Darstellung eines Details aus Fig. 2 ist aus Fig. 3 ersichtlich. Gleiche Bezugszeichen weisen hier wie auch in den weiteren Fig. en auf gleiche oder entsprechende Teile hin.

Wie aus Fig. 3 ersichtlich ist, ist der Unterdruckraum 23 rechteckig und im Beispielsfall nahezu quadratisch. Es versteht sich, dass anstelle dessen auch ein beliebiger anderer, auch ein abgerundeter Unterdruckraum 23 möglich ist. Wie aus Fig. 3 ersichtlich ist, erstreckt sich die Saugdüse 12 als Ringspalt. Die Länge der Saugdüse 12 in Strömungsrichtung ist deutlich größer als die Breite des Ringspalts. In dem dargestellten Ausführungsbeispiel ist die Saugdüse 12 exakt radial einwärts ausgerichtet. Es versteht sich, dass anstelle dessen auch eine Ausrichtung, die hiervon abweicht, günstig sein kann.

So kann die Saugdüse etwas in Richtung intraoral, also in Richtung Folienelement 32, ausgerichtet sein. In diesem Fall ist die Hauptsaugleistung auf den Mundinnenraum des Patienten gerichtet, sodass seine Atemluft umgehend abgesaugt wird.

Es ist aber auch möglich, die Saugdüse 12 nach extraoral schräg gestellt auszurichten, also von dem Folienelement 32 weg weisend. Bei dieser Ausgestaltung wird schwerpunktmäßig die Atemluft vor dem Absaugring 10 abgesaugt.

Die Abweichung von der radialen Erstreckung kann beispielsweise 5 Grad, 10 Grad, aber auch bis zu 45° betragen.

Aus Fig. 3 ist auch ersichtlich, dass der Spannlappen 34 durch die Öse 22 hindurch verläuft. In diesem Ausführungsbeispiel nimmt die Hülse 22 zudem einen Teil des Spannrings 30 auf, so dass dieser dort ebenfalls eingeklemmt wird.

Aus Fig. 4 ist der Einsatz eines erfindungsgemäßen Absaugrings 10 an einem Patienten ersichtlich. Das Folienelement 32 bedeckt die Lippen, wobei das Spannelement 28 im Vestibulum verläuft.

Der Absaugring 10 erstreckt sich extraoral und umgibt den Mund des Patienten, auch wenn dieser geöffnet ist.

Das Saug-Spannelement 18 hält das Folienelement 32 aufgespannt, sodass das Spannelement 30 im Grunde von der technischen Funktionen her entbehrlich ist. Allerdings ist es günstig an, das Spannelement 30 für die Lagerung zwischen dem Halter 26 und dem Saug-Spannelement 18 zu verwenden.

Aus den Fig. en 5 und 6 sind die Luftströme 42 und 44 beim Einsatz des erfindungsgemäßen Absaugrings 10 ersichtlich. Die Luftströmung erfolgt zunächst nach radial auswärts in die Saugdüse 12 hinein, dann durch den Unterdruckraum 23 hindurch und über den Sauganschluss 25 zu einer Unterdruckquelle.

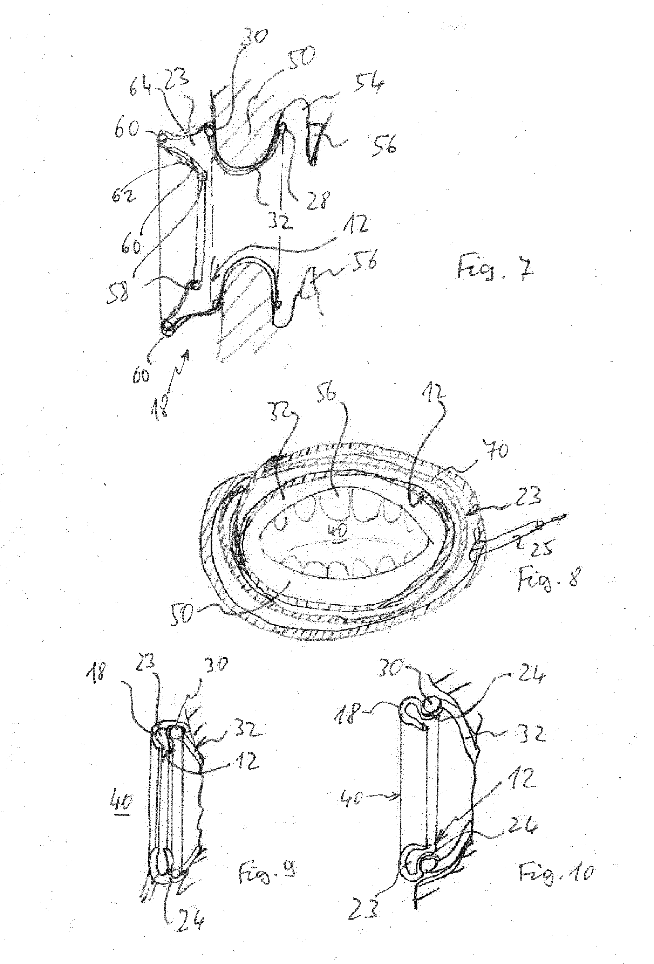

Eine weitere Ausführungsform eines erfindungsgemäßen Absaugrings 10 ist aus Fig. 7 ersichtlich. Das Saug-Spannelement 18 ist bei dieser Ausführungsform von einem umlaufenden Ring gebildet, ähnlich den Spannringen 28/32 des Halters 26. Der Unterdruckraum 23 ist bei dieser Lösung von einer Folie entsprechend der Folie des Fundaments 32 begrenzt.

Die Saugdüse 12 ist zwischen dem vordersten/innersten Ring 58 des Spannelement 18 und dem Folienelement 32 ausgebildet, wenn letzteres an der Lippe 50 des Patienten anliegt. In Fig. 7 sind zudem der Deutlichkeit halber das Vestibulum 54 und Schneidezähne 56 des Patienten schematisch angedeutet.

Auch hier verläuft der Absaugring 10 kreisförmig, und Fig. 7 stellt einen senkrechten Schnitt durch diesen dar, sodass nur ein Teil des Absaugrings 10 im Bereich des Oberkiefers und ein Teil im Bereich des Unterkiefers ersichtlich sind.

Bei der Ausgestaltung gemäß Fig. 7 ist es vorgesehen, das Spannelement 18 aus einem Gebilde aus mehreren Ringen 30, 58 und 60 auszugestalten. Diese Ringe sind über Hilfsstege 62 und 64, die in Fig. 7 schematisch angedeutet sind, miteinander verbunden. Die Verbindung ist elastisch, aber so, dass der Unterdruckraum 23 aufgespannt gehalten wird.

Bei dieser Ausführungsform bilden im Grunde die Ringe 30, 58 und 60 das Saug-Spannelement 18, zusammen mit der Folie 66, die sich zwischen diesen Ringen erstreckt.

Eine demgegenüber modifizierte Ausführungsform ist aus Fig. 8 ersichtlich. Im Vergleich mit Fig. 7 entfallen die Stege 62 und 64. Anstelle dessen ist das Saug-Spannelement 18 durch eine umlaufende Ringspirale 70 gebildet.

Der Unterdruckraum 23 ist auch bei dieser Lösung mit dem Sauganschluss 25 in Verbindung, und die Saugdüse 12 erstreckt sich zwischen dem innersten Ring 58 und dem Folienelement 32.

Diese Ausführungsform hat den Vorteil, dass sie preisgünstig herzustellen ist, mit dem gleichen oder einem entsprechenden Werkzeug, mit dem auch ein "Optragate" herstellbar ist.

Eine weitere erfindungsgemäße Ausführungsform ist aus Fig. 9 ersichtlich. Diese Ausführungsform ist lediglich schematisch dargestellt. Das Folienelement 32 erstreckt sich wie in Fig. 7 dargestellt und ist an dem Spannring 30 befestigt.

Das Saug-Spannelement 18 ist mit Klammern 24 als Aufnahmemittel 20 versehen. Die Klammern 24 übergreifen das Spannelement 30. Sie sind teilringförmig, aber nicht so kurz wie in Fig. 1, sondern erstrecken sich beispielsweise über 45 Grad.

Der Spannring 30 als Teil des Halters 26 ist in die Klammern 26 eingeklipst. hierdurch ist eine sichere Verbindung zum Spannelement 18 hergestellt, aber das Spannelement 18 des Absaugrings 10 ist ohne weiteres lösbar, sodass es wiederverwendbar ist.

Das Spannelement 18 ist als Kunststoffformteil ausgebildet. Es weist eine abgerundete Form auf und bildet den Unterdruckraum 23 und die Saugdüse 12 aus. Diese ist auf die Mitte 40 ausgerichtet.

Eine demgegenüber ähnliche Ausführungsform ist aus Fig. 10 ersichtlich. Auch hier ist das Spannelement 18 als Kunststoffformteil ausgebildet.

Das Spannelement 18 ist wie bei der Ausführungsform gemäß Fig. 9 abgerundet und weist weder Innenecken noch scharfe Kanten auf. Dies kommt der Möglichkeit der Sterilisierung bzw. Reinigung zugute, aber auch der Reduktion der Verletzungsgefahr.

Im Unterschied zu der Ausführungsform gemäß Fig. 9 greifen die Klammern 24 von innen in das Spannelement 30 ein. Sie spreizen es nach außen, aber hintergreifen es auch, so dass eine sichere Lagerung gewährleistet ist.

Auch hier ist der Halter 26 mit dem Folienelement 32 und den Spannelementen 30 und 28 austauschbar und das Saug-Spannelement 18 ist wiederverwendbar.

## Patentansprüche

1. Absaugring für die Anbringung nahe der Mundöffnung eines zahnärztlichen Patienten, mit einem extraoralen Spannelement, **dadurch gekennzeichnet, dass** das Spannelement als Saug-Spannelement, ggf. in Zusammenwirken mit einem Folienelement, einen Unterdruckraum ausbildet oder aufspannt, welcher Unterdruckraum sich ringförmig oder teilringförmig erstreckt, und dass der Unterdruckraum mindestens eine, insbesondere radial einwärts weisende, Saugdüse aufweist.

2. Absaugring nach Anspruch 1, **dadurch gekennzeichnet, dass** das Saug-Spannelement sich ringförmig um die Saugdüse erstreckt und/oder dass die Saugdüse sich ringförmig erstreckt.

3. Absaugring nach dem vorhergehenden Anspruch, **dadurch gekennzeichnet, dass** die Saugdüse einen sich radial erstreckenden Saugspalt oder eine Mehrzahl von sich radial erstreckenden Saugdüsen aufweist.

4. Absaugring nach einem der vorhergehenden Ansprüche, abgesehen von dem vorhergehenden Anspruch, **dadurch gekennzeichnet, dass** die Saugdüse für die Absaugung von Gasen, insbesondere Aerosolen, geeignet ausgebildet ist.

5. Absaugring nach einem der vorhergehenden Ansprüche, **dadurch gekennzeichnet, dass** der Unterdruckraum mit einem Sauganschluss für den Anschluss an eine Unterdruckquelle fest verbunden ist.

6. Absaugring nach einem der vorhergehenden Ansprüche, **dadurch gekennzeichnet, dass** das Saug-Spannelement in der Draufsicht von vorne eine runde, insbesondere eine kreisrunde, eine ovale oder eiförmige Form hat.

7. Absaugring nach einem der vorhergehenden Ansprüche, **dadurch gekennzeichnet, dass** das Saug-Spannelement Aufnahmemittel für das Folienelement aufweist, über welche es mit dem Folienelement, insbesondere lösbar, verbunden oder verbindbar ist.

8. Absaugring nach einem der vorhergehenden Ansprüche, **dadurch gekennzeichnet, dass** Folienelement mit dem Saug-Spannelement fest verbunden ist und/oder sich mindestens teilweise über das Saug-Spannelement erstreckt.

9. Absaugring nach einem der vorhergehenden Ansprüche, **dadurch gekennzeichnet, dass** das Saug-Spannelement das Folienelement im gespannten Zustand hält, und insbesondere durch das Folienelement selbst unter Spannung gesetzt wird.

10. Absaugring nach einem der vorhergehenden Ansprüche, **dadurch gekennzeichnet, dass** das Folienelement für die Erstreckung über die Lippe eines Patienten ausgebildet ist, an welcher Stelle das Folienelement ein Lippen-Abdeckelement bildet.

11. Absaugring nach einem der vorhergehenden Ansprüche, **dadurch gekennzeichnet, dass** das Saug-Spannelement als extraorales Saug-Spannelement ausgebildet ist, das über ein Folienelement als Lippen-Abdeckelement mit einem intraoralen Spannelement verbunden ist.

12. Absaugring nach einem der vorhergehenden Ansprüche, **dadurch gekennzeichnet, dass** das Saug-Spannelement mit einem Lippen/Wangen-Halter verbunden oder verbindbar ist oder mit diesem einstückig ausgebildet ist.

13. Absaugring nach einem der vorhergehenden Ansprüche, **dadurch gekennzeichnet, dass** ein Lippen/Wangen-Halter mit dem erfindungsgemäßen Absaugring nachrüstbar ist.

14. Absaugring nach einem der vorhergehenden Ansprüche, **dadurch gekennzeichnet, dass** der Unterdruckraum und/oder das Saug-Spannelement als formstabiler Kunststoffkörper, insbesondere als Spritzgussteil, ausgebildet ist.

15. Absaugring nach einem der vorhergehenden Ansprüche, **dadurch gekennzeichnet, dass** das Saug-Spannelement durch einen nach radial außen vorgespannten Draht aus Kunststoff oder aus Metall gebildet ist oder diesen aufweist, und/oder, dass das Saug-Spannelement sich spiralig erstreckt, insbesondere in einer Spirale, deren Gänge unterschiedliche Durchmesser aufweisen, und/oder, dass die Saugdüse zwischen dem Abdeckelement und dem Spannelement, insbesondere dem letzten Gang des Spannelements, gebildet ist.

16. Absaugring als Nachrüstteil oder Zubehörteil zu einem Lippen/Wangen-Halter.
